# FASCICULE DE BREVET EUROPEEN

(11) **EP 1 094 791 B1**
(45) Date de publication et mention de la délivrance du brevet: **16.04.2003**
(21) Numéro de dépôt: 99929399.6
(22) Date de dépôt: 05.07.1999
(51) Int. Cl.: A61K 9/48

(54) **FORMULATIONS THIXOTROPES POUR LE REMPLISSAGE DE GELULES**
THIXOTROPE FORMULIERUNGEN ZUR BEFÜLLUNG VON GELATINEKAPSELN
THIXOTROPIC FORMULATIONS FOR FILLING CAPSULES

(30) Priorité: 07.07.1998 FR 9808664
(43) Date de publication de la demande: 02.05.2001
(73) Titulaire: PIERRE FABRE MEDICAMENT, 92100 Boulogne-Billancourt (FR)
(72) Inventeur: LOMBARDIN, Pascal, F-94410 Saint-Maurice (FR); GROSSIORD, Jean-Louis, F-91250 Saint-Germain-les-Corbeil (FR); SEILLER, Monique, F-92330 Sceaux (FR); LEVERD, Elie, F-81100 Castres (FR); GOUTAY, Eric, F-31650 Lauzerville (FR); BOUGARET, Jo[l, F-31570 Lanta (FR)
(74) Mandataire: Ahner, Francis
(86) Numéro de dépôt international: FR9901614
(87) Numéro de publication internationale: WO00001371

(56) Documents cités:
- EP-A- 0 461 290
- EP-A- 0 517 412
- WO-A-96/41622
- FR-A- 2 365 338
- FR-A- 2 761 265

## Description

La présente invention concerne des compositions pharmaceutiques ou vétérinaires, diététiques ou cosmétiques thixotropes contenant une ou plusieurs substances actives, destinées au remplissage à température ambiante, de capsules à enveloppe dure appelées gélules.

On entend par température ambiante, une température sensiblement comprise entre 15 et 30°C.

Deux types de capsules sont utilisés pour les médicaments destinés à l'administration orale, rectale ou vaginale: les capsules à enveloppe molle et les capsules à enveloppe dure.

Les compositions pharmaceutiques unitaires liquides ou pâteuses sont traditionnellement présentées en capsules molles. Cependant, le procédé de fabrication des capsules molles nécessite le recours à des installations complexes et à des façonniers spécialisés, si bien que l'utilisation de gélules peut, pour des raisons économiques, lui être préférée.

Les gélules sont, quant à elles, traditionnellement utilisées pour le conditionnement des substances solides comme les poudres et les granulés. Dans certains cas, le remplissage des gélules avec des substances solides présente certains problèmes techniques tels que, d'une part, la génération de poussières contaminantes lors de la manipulation de substances actives et toxiques (anticancéreux, hormones) - ce qui peut s'avérer particulièrement dangereux - et, d'autre part, le remplissage non uniforme d'une gélule à l'autre lorsque la ou les substances actives sont faiblement dosées.

C'est pourquoi, une substance active solide peut être associée à un véhicule liquide avant d'être conditionnée en gélules (US H 672).

L'utilisation d'un véhicule liquide pour le remplissage de gélules soulève également des problèmes, car le liquide peut s'écouler entre le corps et la tète de la gélule. Les fuites sont généralement évitées par scellage des gélules (EP-488 181 et WO-91/02520). Cette opération de scellage nécessite un savoir-faire particulier et une étape supplémentaire entraînant un surcoût non négligeable.

Une alternative au scellage des gélules a été proposée. Elle consiste à remplir les gélules avec une composition contenant le principe actif à l'état dissous ou dispersé. Cette même composition est liquide ou pâteuse et de faible viscosité lors du remplissage et s'épaissit ensuite à l'intérieur des gélules.

Selon un premier mode de remplissage, dit "remplissage à chaud", la composition, qui est pâteuse à température ambiante, est fluidifiée par chauffage (EP-49 909). Cette méthode ne peut pas s'appliquer à des principes actifs thermo-sensibles comme certains anticancéreux, vitamines et antibiotiques.

Selon un deuxième mode de remplissage, dit "remplissage à température ambiante", GB-1 590 864 propose des compositions telles que
- leur viscosité à 20 ± 1°C est comprise entre 500 et 5 000 mPa.s, de préférence entre 1 000 et 3 000 mPa.s, mesurée à 450 tours par minute sur un viscosimètre Haake,
et telles que,
- leur tension de surface est supérieure à 20 dynes/cm, de préférence à 30 dynes/cm.

GB-1 590 864 ne précise pas cependant quelle doit être la viscosité des compositions au repos.

Par ailleurs, un troisième mode de remplissage combinant les deux premiers a été décrit dans EP-49 909. Selon cette méthode, on chauffe à 40°C une composition rhéofluidifiante contenant de la paraffine liquide, de l'huile de ricin hydrogénée et de la silice colloïdale.

La Demanderesse a démontré que le caractère rhéofluidifiant des compositions pour remplissage de gélules de l'art antérieur, bien que nécessaire pour assurer un bon remplissage de la gélule, se révèle néanmoins insuffisant.

Il est, en effet, également impératif de vérifier que la formulation au repos dans la gélule, se restructure de façon suffisamment intense et surtout suffisamment rapide, après le remplissage, pour éviter toute fuite entre les deux parties de la gélule.

Il est donc indispensable que la consistance de la composition au repos soit suffisante pour éviter tout écoulement de la composition entre les deux parties de la gélule.

Parmi les matières premières utilisées dans les compositions de remplissage classiques figurent les polyéthylèneglycols. Les polyéthylèneglycols sont destinés à dissoudre le principe actif hydrosoluble de la composition de remplissage, grâce à leurs propriétés hydrophiles (EP-276 116, EP-488 181 et EP-49 909).

On a constaté que l'incorporation, en tant que phase continue, de polyéthylèneglycols, et plus particulièrement de polyéthylèneglycols de basses masses moléculaires moyennes, dans la composition de remplissage peut poser de sérieux problèmes d'interactions physico-chimiques et donc de stabilité.

En particulier, ces polyéthylèneglycols sont hygroscopiques et attirent l'eau de la gélatine dans la phase continue rendant l'enveloppe cassante et fragile au cours du stockage.

Les compositions de remplissage de la présente invention sont avantageusement dépourvues de tout polyéthylènegtycol et, en particulier, de polyéthylèneglycol de basse masse moléculaire moyenne qui risquerait de fragiliser l'enveloppe des gélules.

La présente invention a, pour objectif, de fournir des compositions qualifiées de "thixotropes" contenant une ou plusieurs substances actives qui permettent un remplissage aisé des gélules à température ambiante, et qui garantissent l'absence de fuites entre les deux parties de la gélule sans qu'il soit nécessaire d'avoir recours au scellage habituellement préconisé pour ce type de forme galénique.

Les propriétés rhéologiques des formulations de l'invention garantissent un remplissage efficace à température ambiante et une absence de fuite des gélules remplies.

On rappellera qu'une composition thixotrope liquide ou pâteuse possède un caractère rhéofluidifiant qui se manifeste par une diminution de la viscosité apparente sous l'effet d'un cisaillement croissant. En outre, toute variation des conditions de cisaillement provoque une modification structurelle différée dans le temps. C'est ainsi qu'on observe en particulier une reprise progressive, totale ou partielle, de la consistance après l'arrêt du cisaillement.

Les grandeurs rhéologiques choisies comme particulièrement représentatives de la consistance des formulations sont :
- le module complexe G* dont la valeur est d'autant plus importante que le produit étudié est consistant et qui fait la synthèse des propriétés élastiques et visqueuses du matériau, et
- le déphasage δ compris entre 0° et 90°, sachant qu'un déphasage supérieur à 45° caractérise une prédominance visqueuse et qu'inversement, un déphasage inférieur à 45° met en évidence une prédominance élastique caractéristique d'un matériau structuré.

La présente invention concerne des compositions thixotropes liquides ou pâteuses contenant une ou plusieurs substances actives destinées au remplissage de gélules à température ambiante, telles que :
- leur module complexe G* est supérieur à environ 100 Pa,
- leur déphasage δ est inférieur à environ 45°,
- leur viscosité diminue à gradient de cisaillement croissant,
- sous l'effet d'un gradient de cisaillement constant γₒ, la viscosité desdites compositions diminue de façon différée dans le temps, et se stabilise à la valeur d'équilibre η_{eq} comprise entre 10 mPa.s et 10 000 mPa.s environ, lorsque γₒ est compris entre 100 et 1 000 s⁻¹, et
- après arrêt dudit gradient de cisaillement, le module complexe et le déphasage desdites compositions retrouvent, au bout d'une durée t inférieure à 1 heure des valeurs G* et de δ respectivement supérieures à environ 100 Pa et inférieures à environ 45°.

Les compositions selon l'invention sont donc définies, d'une part, par leur caractère rhéofluidifiant, c'est-à-dire que leur viscosité diminue lorsque l'intensité du cisaillement croît, et, d'autre part, par la diminution de leur viscosité au cours du temps pour un cisaillement donné.

Les formulations de l'invention se fluidifient ainsi dans la machine de remplissage des gélules sous l'effet du cisaillement induit par l'agitation présente depuis la trémie d'alimentation jusqu'à la buse de répartition. Cette propriété rend particulièrement aisé le remplissage des gélules.

Pour chaque gradient de cisaillement, la viscosité des compositions de l'invention diminue au cours du temps et se stabilise finalement à une valeur d'équilibre notée η_{eq}. Les compositions selon l'invention présentent des viscosités à l'équilibre η_{eq} à 100 s⁻¹ et 1 000 s⁻¹ comprises entre 10 mPa.s et 10 000 mPa.s, préférentiellement entre 100 mPa.s et 1 500 mPa.s. Il n'est nullement nécessaire de recourir à une opération de chauffage, comme l'imposent certains procédés de l'art antérieur (US-4,450,877).

Les compositions selon l'invention sont également définies par une reprise de consistance importante différée dans le temps.

Les compositions de l'invention, fluidifiées dans la machine de remplissage des gélules, retrouvent leur consistance initiale après un temps de repos suffisant, de façon à éviter tout risque de fuite au niveau de la gélule pleine.

Les formulations selon l'invention sont caractérisées par des valeurs de G* supérieures à 100 Pa, de préférence à 1 000 Pa, et/ou des valeurs de δ inférieures à 45°, de préférence inférieures à 25°, et/ou un temps de reprise t inférieur à 1 heure et de préférence inférieur à 30 minutes, et/ou des valeurs de η_{eq} comprises entre 100 mPa.s et 1500 mPa.s lorsque le gradient de cisaillement est compris entre 100 et 1 000 s⁻¹.

Une fois la reprise achevée, G*_{eq} est supérieur à 100 Pa, préférentiellement 1 000 Pa, et δ_{eq} est inférieure à 45°, préférentiellement à 25°.

Les gélules utilisées dans le cadre de la présente invention sont constituées de gélatine, d'un polymère cellulosique (comme l'hydroxypropylméthylcellulose) ou de tout autre polymère capable d'assurer les fonctions d'usage de la gélatine sous la forme de gélule.

Selon un mode de réalisation préféré, les compositions thixotropes de la présente invention sont des dispersions contenant une phase continue dispersante liquide ou pâteuse, une phase dispersée à l'état particulaire ou micellaire, modulatrice de la viscosité, et au moins une substance active présente sous la forme dissoute et/ou dispersée.

Les phases dispersantes de l'invention sont caractérisées par leur large éventail de polarité en terme de balance hydrophile-lipophile (HLB). Les matières premières qui entrent dans la constitution de ces phases dispersantes de l'invention présentent des propriétés hydrophiles, lipophiles ou amphiphiles d'HLB variable qui permettent la dissolution ou la dispersion de principes actifs liquides et solides, eux-mêmes hydrophiles, lipophiles ou amphiphiles.

La phase continue de ces compositions est avantageusement constituée d'au moins un véhicule tel que des huiles, leurs dérivés, et plus particulièrement des esters amphiphiles présentant un HLB compris entre 3 et 15, tels que des glycérides polyglycolisés amphiphiles, comme les Labrasol® et Labrafil® commercialisés par la Société GATTEFOSSE.

L'utilisation de véhicules amphiphiles à tendance hydrophile représente une bonne alternative aux polyéthylèneglycols hydrophiles de l'art antérieur. Hormis les polyéthylèneglycols, les produits qui entrent classiquement dans la constitution de préparations thixotropes liquides ou pâteuses pour gélule sont plutôt lipophiles (GB-1 590 864, US-4,450,877, US-H672, EP-461 290).

Les phases continues amphiphiles à tendance hydrophile utilisées dans le cadre de la présente invention s'avèrent, contrairement aux excipients de l'art antérieur, parfaitement adaptées aux principes actifs hydrophiles, lipophiles ou amphiphiles qu'elles solubilisent ou dispersent respectivement.

La phase dispersée modulatrice de la viscosité des compositions selon l'invention peut être choisie parmi des particules de silice pyrogénée hydrophile ou hydrophobe dont la taille moyenne peut être comprise entre 5 et 50 nm préférentiellement entre 7 et 20 nm et la surface spécifique comprise entre 10 et 450 m²/g préférentiellement entre 70 et 410 m²/g, comme l'Aérosil® commercialisé par la société DEGUSSA, et des copolymères d'oxyde d'éthylène et d'oxyde de propylène, comme les Synpéronic® commercialisés par la Société ICI, et de leurs mélanges.

La phase dispersée en association avec la phase continue permet d'atteindre des HLB allant jusqu'à environ 20.

La phase dispersée modulatrice de la viscosité des compositions selon l'invention représente de préférence 1 à 30 % m/m, de préférence encore 5 à 15 % m/m, de la préparation.

Les excipients entrant dans la formulation des compositions thixotropes selon l'invention sont choisis parmi les excipients pharmaceutiquement acceptables et inertes vis-à-vis des substances actives que l'on souhaite formuler.

En outre, ces excipients sont choisis parmi les excipients compatibles avec la tunique des gélules.

Les excipients qui entrent dans la constitution des compositions thixotropes selon l'invention sont avantageusement dotés de propriétés hydrophiles, lipophiles ou amphiphiles, avec pour ces derniers une balance hydrophile, lipophile (HLB) variable, qui permettent la dissolution ou la dispersion de substances actives aussi bien hydrophiles que lipophiles. Le HLB des véhicules peut varier de 4 ± 1, pour une association de Labrafil® M1944CS et d'Aérosil®, à 20 ± 1, pour une association de Labrasol® et de Synpéronic®.

Les compositions selon l'invention contiennent une substance active qui peut être liquide, pâteuse mais aussi solide, par exemple le chlorhydrate de milnacipran (solubilité dans l'eau égale à 600 g/l), le baquimast (solubilité dans l'eau égale à 0,23 g/l), la nifédipine, le triamtérène, l'hydroxychlorure d'aluminium, le salicylate de sodium, la vancomycine, la paraméthadone et la griséofulvine.

Les gélules utilisées dans le cadre de la présente invention sont constituées de gélatine ou de tout autre polymère cellulosique, capable de remplir les fonctions d'usage de la gélatine sous la forme d'une gélule, telle que l'hydroxypropylméthylcellulose.

L'invention ne se limite pas à ces exemples et l'homme du métier pourra aisément inclure dans les compositions décrites toute substance active de son choix, qu'elle soit liquide, pâteuse ou même solide.

La présente invention concerne également l'utilisation des compositions décrites précédemment dans une préparation cosmétique, diététique, pharmaceutique ou vétérinaire.

La présente invention est illustrée par les exemples suivants en référence aux figures annexées :
- la figure 1 représente le rhéogramme d'une formulation de l'exemple 4 de l'invention et le rhéogramme d'une composition de l'exemple 5 dont les propriétés rhéologiques ne répondent pas aux critères de l'invention.
   La contrainte est donnée en ordonnée (en Pascal) et le gradient de cisaillement (en s⁻¹) est donné en abscisse.
- La figure 2 donne la cinétique de reprise de consistance de deux formulations de l'invention, celle de l'exemple 2 et celle de l'exemple 4.
   Le module complexe exprimé en Pascal est donné en ordonnée et le temps est donné en abscisse.
- Les figures 3 et 4 représentent le pourcentage de dissolution (en ordonnée) respectivement d'une formulation de l'exemple 1 et d'une formulation de l'exemple 2, en fonction du temps (en abscisse), respectivement exprimé en heures et en minutes.

### EXEMPLES 1 A 7 :

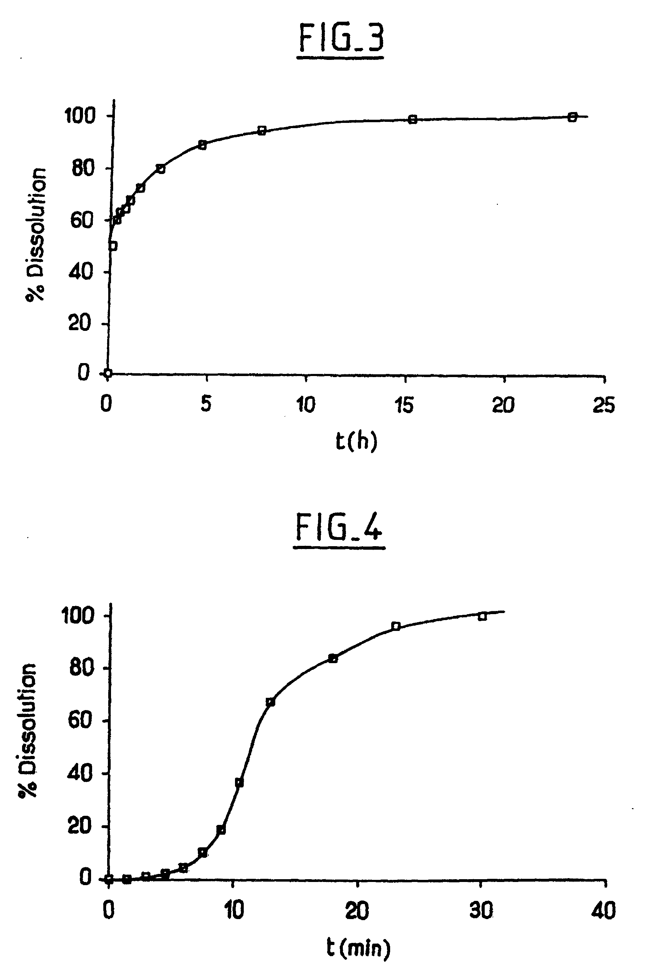

**a) Préparation des dispersions :**
   On prépare sept dispersions contenant chacune une phase continue, une phase dispersée et une substance active.
   La phase continue est constituée d'un ester amphiphile comme le Labrafil M1944CS® (HLB = 4 ± 1) ou le Labrasol® (HLB = 14 ± 1). On mentionnera à ce stade que les esters amphiphiles utilisables dans le cadre de l'invention peuvent présenter des HLB compris entre 3 et 15.
   La phase dispersée est choisie parmi l'Aérosil 200 V® (silice pyrogénée hydrophile), l'Aérosil R 974® (silice pyrogénée hydrophobe) et le Synpéronic PE/F 68® (copolymère d'oxyde d'éthylène et d'oxyde de propylène d'HLB égal à 29 ± 1). Lorsque la phase dispersée choisie est le Synpéronic®, le HLB de la phase dispersante passe à 20 environ.
   La substance active est choisie parmi le chlorhydrate de milnacipran (solide dont la solubilité dans l'eau est égale à 600 g/l) et le baquimast (solide dont la solubilité dans l'eau est égale à 0,23 g/l).
   Les préparations contenant de la silice pyrogénée comme phase dispersée sont obtenues par ajout progressif de la silice à l'ester amphiphile, sous agitation intense avantageusement comprise entre 1 000 et 3 000 tours par minute. Le mélange est ensuite placé sous vide et l'agitation est maintenue après incorporation de la silice jusqu'à homogénéité. Les préparations contenant du Synpéronic® comme phase dispersée sont obtenues par ajout progressif de Synperonic® à l'ester amphiphile, sous agitation modérée avantageusement comprise entre 400 et 800 tours par minute. Le mélange est ensuite placé sous vide et l'agitation est maintenue jusqu'à homogénéité.
   Que les préparations contiennent de l'Aérosil® ou du Synperonic® comme phase dispersée modulatrice de la viscosité, la substance active choisie est toujours ajoutée au mélange ester amphiphile/phase dispersée, à température ambiante et sous agitation modérée. La composition de chaque dispersion est détaillée dans le tableau I ci-après. 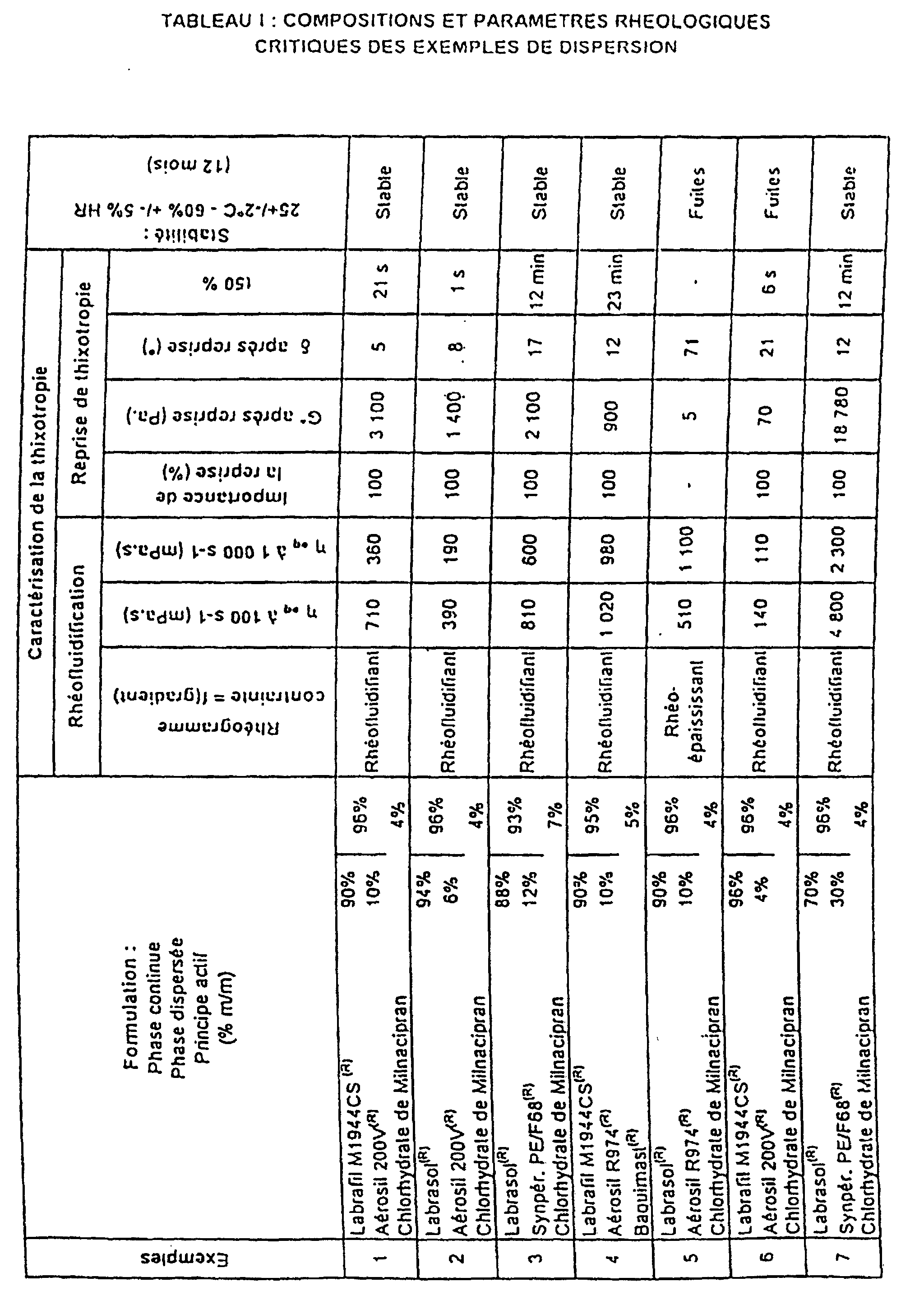
**b) Propriétés rhéologiques**
   Les propriétés rhéologiques des sept préparations sont ensuite étudiées, en termes de rhéofluidification et de reprise de consistance.
   b1) La rhéofluidification a été caractérisée à 25°C en rhéologie d'écoulement sur un rhéomètre rotatif à contrainte imposée (Carri-Med CSL100).
      Le rhéogramme "contrainte en fonction du gradient de cisaillement" est tracé pour. chacune des dispersions. Le rhéogramme permet de vérifier l'aptitude d'une préparation à se fluidifier lorsque l'intensité du cisaillement croît.
      Puisque la viscosité se définit comme étant le rapport contrainte/gradient, l'obtention d'une courbe convexe exprime une diminution de la viscosité avec le gradient de cisaillement, c'est-à-dire un comportement rhéofluidifiant, tandis que l'obtention d'une courbe concave exprime une augmentation de la viscosité avec le gradient de cisaillement, c'est-à-dire un comportement rhéo-épaississant.

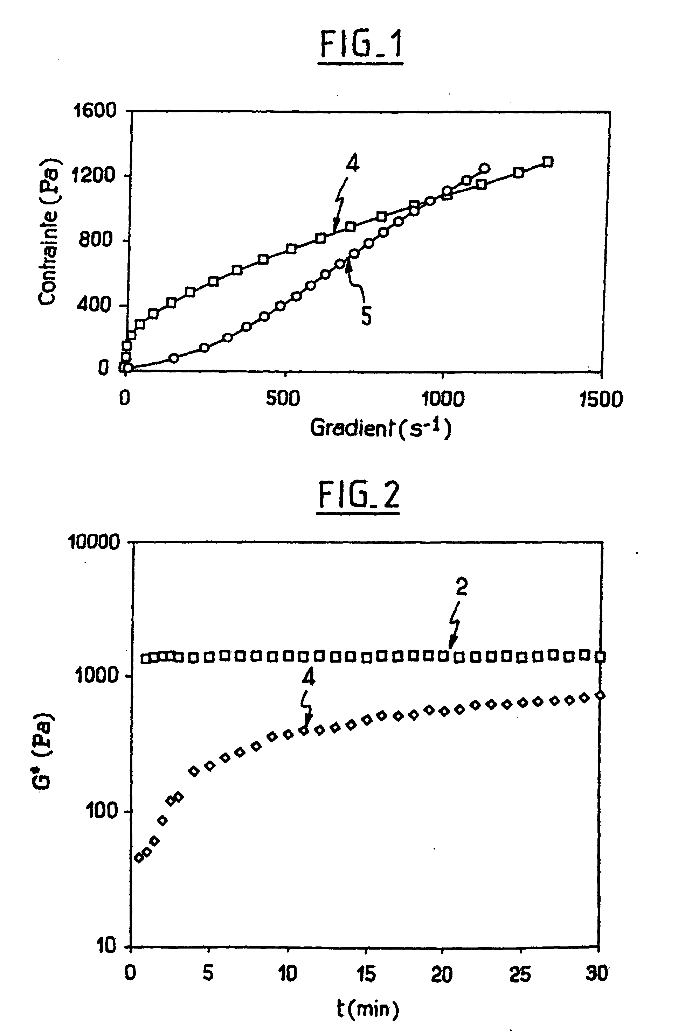

      La figure 1 représente le rhéogramme de deux dispersions, la dispersion 4 et la dispersion 5 dont les compositions sont rappelées dans le tableau I ci-dessus. Le tracé du rhéogramme de la dispersion 4 qui est convexe indique que la dispersion 4 est rhéofluidifiante et remplit un des critères des compositions de l'invention, tandis que celui de la préparation 5 qui est concave prouve son caractère rhéo-épaississant.
      Le tableau I précise le caractère rhéofluidifiant ou rhéo-épaississant constaté au vu du tracé du rhéogramme de chaque dispersion.
      Les formulations sont également soumises à des gradients de cisaillement constants du même ordre de grandeur que ceux mis en oeuvre au niveau d'une machine de remplissage de gélules de type classique (100 s⁻¹ pour les canalisations de la machine, 1 000 s⁻¹ pour le rétrécissement à la sortie de la buse d'injection).
      On constate pour chaque gradient de cisaillement une diminution de la viscosité au cours du temps qui se stabilise finalement à une valeur d'équilibre notée η_{eq}. Les résultats sont présentés dans le tableau I.
      Les sept dispersions présentent des viscosités à l'équilibre, à un gradient 100 s⁻¹ ou 1 000 s⁻¹, comprises entre 100 mPa.s et 5 000 mPa.s.
      Ces valeurs de viscosité s'avèrent par conséquent adaptées au remplissage automatique des gélules. Il n'est nullement nécessaire de fluidifier davantage nos dispersions thixotropes en élevant la température de remplissage comme le préconisent les auteurs du brevet US-4 450 877.
   b2) Les conditions rhéologiques relatives à la reprise de thixotropie et qui garantissent l'absence de fuites à long terme ont été déterminées à 25°C en rhéologie dynamique sur rhéomètre rotatif de type Couette à contrainte imposée (Carri-Med CSL100).
      Ce mode d'étude permet, contrairement à la rhéologie d'écoulement, d'apprécier la consistance d'un matériau "au repos" puisqu'il est possible d'appliquer au matériau des déformations notablement plus faibles qu'en écoulement.
      Les sept dispersions sont préalablement soumises pendant 15 min. à un cisaillement de 1 000 s⁻¹.
      Les paramètres retenus pour caractériser la reprise de consistance sont l'importance de la reprise (exprimée en pourcentage), le module complexe G* après la reprise (en Pascal) et le déphasage δ 1 heure après la suppression du cisaillement (en degré) ainsi que le temps t_{50 %} au bout duquel la reprise est achevée à 50 % par rapport à G*_{eq}. Les résultats sont résumés dans le tableau I ci-dessus.
      Le pourcentage de reprise de consistance des dispersions rhéofluidifiantes 1, 2, 3, 4, 6 et 7 est égal à 100 %. La reprise est donc totale. Les valeurs de la reprise G* après 1 heure sont totales et supérieures à 100 Pa pour les préparations 1 à 4 et 7, mais inférieures à 100 Pa pour les préparations 5 et 6 atteignant respectivement les valeurs de 5 et 70 Pa.
      δ après la reprise est inférieur à 25° pour toutes les dispersions à l'exception de la dispersion 5 (égal à 71°).
      t_{50%} est inférieur à 30 min. pour les cinq dispersions rhéofluidifiantes.
      Les dispersions 1, 2, 3, 6 et 7, présentent donc une reprise rapide et totale de leur consistance qui est, en outre, importante.
      La figure 2 donne l'évolution de G* en fonction du temps pour les dispersions 2 et 4. On constate que la reprise de consistance de la dispersion 2 est très rapide (t_{50 %} = 1 s) et importante (G* après reprise = 1 400 Pa), celle de la dispersion 4 est plus lente (t_{50 %} = 23 min.) et importante (G* après reprise = 900 Pa).
**c) Etude de stabilité des gélules**
   Une fois les propriétés rhéologiques des dispersions étudiées, les dispersions sont réparties en gélules de gélatine à fermeture classique de taille 1 à température ambiante à l'aide d'une machine de remplissage de type industriel. Le remplissage s'effectue avec un coefficient de variation sur la masse de la gélule remplie systématiquement inférieur à 1,5 %.
   Les gélules remplies sont stockées pendant 12 mois à l'étuve (25°C ± 2°C - 60 % HR ± 5 % HR) pour vérifier l'absence de fuites et de déformations au niveau de la tunique de la gélule. Seules les gélules contenant les préparations 5 et 6 présentent des fuites durant le stockage.
**d) Etude de libération in vitro des gélules**
   Des études de libération in vitro réalisées en Dissolutest (eau à 37 ± 0,5°C, 100 tpm) ont été menées. Les résultats des préparations 1 et 2 apparaissent respectivement au niveau des figures 3 et 4.
   La libération du chlorhydrate de milnacipran (principe actif solide et hydrosoluble) est d'autant plus rapide que la phase continue est hydrophile.
   Pour les conditions opératoires retenues, la préparation 2 (figure 4) contenant un ester amphiphile à tendance hydrophile (HLB = 14) libère le principe actif beaucoup plus rapidement que la préparation 1 (figure 3) contenant un ester amphiphile à tendance lipophile (HLB = 4 ± 1).
   Le choix de la base continue permet donc d'adapter la vitesse de libération du principe actif.

## Revendications

1. Compositions thixotropes liquides ou pâteuses contenant une ou plusieurs substances actives destinées au remplissage de gélules à température ambiante comprise entre 15 et 30°C, telles que :
• leur module complexe G* est supérieur à environ 100 Pa,
• leur déphasage δ est inférieur à environ 45°,
• leur viscosité diminue à gradient de cisaillement croissant,
• sous l'effet d'un gradient de cisaillement constant γₒ, la viscosité desdites compositions diminue de façon différée dans le temps, et se stabilise à la valeur d'équilibre η_{eq} comprise entre 10 mPa.s et 10 000 mPa.s environ, lorsque γₒ est compris entre 100 et 1 000 s⁻¹, et
• après arrêt dudit gradient de cisaillement, le module complexe et le déphasage desdites compositions retrouvent, au bout d'une durée t inférieure à 1 heure des valeurs de G* et de δ respectivement supérieures à environ 100 Pa et inférieures à environ 45°.

2. Compositions selon la revendication 1, **caractérisées en ce que** :
G* est supérieur à 1 000 Pa, et/ou
δ est inférieur à 25° et/ou
η_{eq} est compris entre 100 et 1 500 mPa.s, lorsque γₒ est compris entre
100 et 1 000 s⁻¹, et/ou
t est inférieur à 30 min.

3. Compositions selon la revendication 1 ou 2, **caractérisées en ce que** ce sont des préparations contenant une phase continue dispersante, une phase dispersée et au moins une substance active.

4. Compositions selon la revendication 3, **caractérisées en ce que** la phase continue est constituée d'au moins un véhicule tel que des esters amphiphiles présentant un HLB compris entre 3 et 15, et plus particulièrement les glycérides polyglycolisés.

5. Compositions selon la revendication 3 ou 4, **caractérisées en ce que** la phase dispersée est choisie parmi des particules de silice pyrogénée hydrophile ou hydrophobe, et des copolymères d'oxyde d'éthylène et d'oxyde de propylène, ces derniers permettant d'atteindre, en association avec la phase continue, des HLB allant jusqu'à environ 20.

6. Compositions selon l'une des revendications 3 à 5, **caractérisées en ce que** la substance active est liquide, pâteuse ou solide.

7. Compositions selon la revendication 6, **caractérisées en ce que** la substance active est choisie parmi le chlorhydrate de milnacipran, le baquimast, la nifédipine, le triamtérène, l'hydroxychlorure d'aluminium, le salicylate de sodium, la vancomycine, la paraméthadone et la griséofulvine.

8. Compositions selon l'une des revendications 3 à 7, **caractérisées en ce que** la phase dispersée des préparations selon l'invention représente 1 à 30 % m/m de la préparation.

9. Compositions selon la revendication 8, **caractérisées en ce que** la phase dispersée des dispersions selon l'invention représente 5 à 15 % m/m de la préparation.

10. Compositions selon l'une des revendications précédentes, **caractérisées en ce que** les gélules sont constituées de gélatine ou de tout autre polymère cellulosique, capable de remplir les fonctions d'usage de la gélatine sous la forme d'une gélule, tel que l'hydroxypropylméthylcellulose.

## Patentansprüche

1. Flüssige oder pastöse thixotrope Zusammensetzungen, die einen oder mehrere Wirkstoffe enthalten und zum Befüllen von Kapseln bei Umgebungstemperatur zwischen 15 und 30°C einschließlich bestimmt sind, derart dass:
■ ihr komplexer Modul G* über etwa 100 Pa liegt,
■ ihre Phasenverschiebung δ unter etwa 45° liegt,
■ ihre Viskosität bei zunehmendem Scherkraftgradienten abnimmt,
■ unter der Einwirkung eines konstanten Scherkraftgradienten γ₀ die Viskosität der Zusammensetzungen auf zeitlich verzögerte Weise abnimmt und sich bei einem Gleichgewichtswert η_{Äq} von etwa 10 mPa·s bis 10.000 mPa·s stabilisiert, wenn γ₀ zwischen 100 und 1.000 s⁻¹ einschließlich liegt, und
■ nach Stoppen des Scherkraftgradienten der komplexe Modul und die Phasenverschiebung der Zusammensetzungen am Ende einer Dauer t von weniger als einer Stunde wieder Werte von G* und δ annehmen, die über etwa 100 Pa bzw. unter etwa 45° liegen.

2. Zusammensetzungen nach Anspruch 1, **dadurch gekennzeichnet, dass**:
G* über 1.000 Pa liegt und/oder
δ unter 25° liegt und/oder
η_{Äq} zwischen 100 und 1.500 mPa·s einschließlich liegt, wenn γ₀ zwischen
100 und 1.000 s⁻¹ einschließlich liegt, und/oder
t unterhalb 30 min liegt.

3. Zusammensetzungen nach Anspruch 1 oder 2, **dadurch gekennzeichnet, dass** es sich um Präparate handelt, die eine kontinuierliche dispergierende Phase, eine dispergierte Phase und mindestens einen Wirkstoff enthalten.

4. Zusammensetzungen nach Anspruch 3, **dadurch gekennzeichnet, dass** die kontinuierliche Phase aus mindestens einem Vehikel, wie amphiphilen Estern, die ein HLB zwischen 3 und 15 einschließlich aufweisen, und spezieller polyglycolisierten Glyceriden, zusammengesetzt ist.

5. Zusammensetzungen nach Anspruch 3 oder 4, **dadurch gekennzeichnet, dass** die dispergierte Phase ausgewählt ist aus pyrogenen hydrophilen oder hydrophoben Siliciumdioxid-Teilchen und Copolymeren von Ethylenoxid und Propylenoxid, wobei die Letztgenannten es ermöglichen, in Verbindung mit der kontinuierlichen Phase HLBs zu erreichen, die bis zu etwa 20 gehen.

6. Zusammensetzungen nach einem der Ansprüche 3 bis 5, **dadurch gekennzeichnet, dass** der Wirkstoff flüssig, pastös oder fest ist.

7. Zusammensetzungen nach Anspruch 6, **dadurch gekennzeichnet, dass** der Wirkstoff aus Milnacipran-Hydrochlorid, Baquimast, Nifedipin, Triamteren, Aluminiumhydroxychlorid, Natriumsalicylat, Vancomycin, Paramethadon und Griseofulvin ausgewählt ist.

8. Zusammensetzungen nach einem der Ansprüche 3 bis 7, **dadurch gekennzeichnet, dass** die dispergierte Phase der erfindungsgemäßen Präparate 1 bis 30% Gew./Gew. des Präparats ausmacht.

9. Zusammensetzungen nach Anspruch 8, **dadurch gekennzeichnet, dass** die dispergierte Phase der erfindüngsgemäßen Dispersionen 5 bis 15% Gew./Gew. des Präparats ausmacht.

10. Zusammensetzungen nach einem der vorangehenden Ansprüche, **dadurch gekennzeichnet, dass** die Kapseln aus Gelatine oder jedem anderen Cellulose-Polymer zusammengesetzt sind, welche die Funktionen der Verwendung von Gelatine in Form einer Kapsel ausüben können, wie Hydroxypropylmethylcellulose.

## Claims

1. Liquid or pasty thixotropic compositions containing one or more active substances, intended for filling hard capsules at room temperature between 15 and 30°C, such that:
• their complex modulus G* is greater than about 100 Pa,
• their phase shift δ is less than about 45°,
• their viscosity decreases with increasing shear rate,
• under the effect of a constant shear rate γ₀, the viscosity of the said compositions decreases in a delayed manner over time and stabilizes at the equilibrium value η_{eq} of between 10 mPa.s and about 10 000 mPa.s, when γ₀ is between 100 and 1000 s⁻¹ and
• after making the said shear rate 0, the complex modulus and the phase shift of the said compositions resume, after a time t of less than 1 hour, G* and δ values of greater than about 100 Pa and of less than about 45°, respectively.

2. Compositions according to Claim 1, **characterized in that**:
G* is greater than 1000 Pa, and/or
δ is less than 25° and/or
η_{eq} is between 100 and 1500 mPa.s when γ₀ is between 100 and 1000 s⁻¹ and/or
t is less than 30 min.

3. Compositions according to Claim 1 or 2, **characterized in that** they are preparations containing a continuous dispersing phase, a dispersed phase and at least one active substance.

4. Compositions according to Claim 3, **characterized in that** the continuous phase consists of at least one vehicle such as amphiphilic esters having an HLB of between 3 and 15 and more particularly polyglycolized glycerides.

5. Compositions according to Claim 3 or 4, **characterized in that** the dispersed phase is chosen from hydrophilic or hydrophobic pyrogenic silica particles and ethylene oxide/propylene oxide copolymers, the latter making it possible to achieve, when combined with the continuous phase, HLB values ranging up to about 20.

6. Compositions according to one of Claims 3 to 5, **characterized in that** the active substance is liquid, pasty or solid.

7. Compositions according to Claim 6, **characterized in that** the active substance is chosen from milnacipran hydrochloride, baquimast, nifedipine, triamterene, aluminium hydroxychloride, sodium salicylate, vancomycin, paramethadone and griseofulvin.

8. Compositions according to one of Claims 3 to 7, **characterized in that** the dispersed phase of the preparations according to the invention represents 1 to 30% m/m of the preparation.

9. Compositions according to Claim 8, **characterized in that** the dispersed phase of the dispersions according to the invention represents from 5 to 15% m/m of the preparation.

10. Compositions according to one of the preceding claims, **characterized in that** the hard capsules consist of gelatin or of any other cellulose polymer capable of fulfilling the functions of the use of gelatin in the form of a hard capsule, such as hydroxypropylmethyl cellulose.
